(19) Europäisches Patentamt
European Patent Office
Office européen des brevets

(11) Publication number: **0 068 173 B1**

# (12) EUROPEAN PATENT SPECIFICATION

(45) Date of publication of patent specification: **26.09.84**

(51) Int. Cl.[3]: **C 07 C 103/52,** A 61 K 31/02

(21) Application number: **82104925.1**

(22) Date of filing: **04.06.82**

## (54) Perhydro-1,4-thiazepin-5-one and perhydro-1,4-thiazocin-5-one derivatives, process for preparing and pharmceutical composition containing the same.

(30) Priority: **05.06.81 US 270756**
**06.04.82 US 364481**

(43) Date of publication of application:
**05.01.83 Bulletin 83/01**

(45) Publication of the grant of the patent:
**26.09.84 Bulletin 84/39**

(84) Designated Contracting States:
**AT BE CH DE FR GB IT LI LU NL SE**

(56) References cited:
**EP-A-0 040 332**
**EP-A-0 046 289**
**EP-A-0 046 291**
**EP-A-0 046 292**

(73) Proprietor: **MERCK & CO. INC.**
**126, East Lincoln Avenue P.O. Box 2000**
**Rahway New Jersey 07065 (US)**

(72) Inventor: **Patchett, Arthur A.**
**1090, Minisink Way**
**Westfield New Jersey 07090 (US)**
Inventor: **Thorsett, Eugene D.**
**4, Poplar Place**
**Fanwood New Jersey 07023 (US)**
Inventor: **Cheung, Yak-Fa**
**909 Harding Street**
**Westfield New Jersey 07090 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of Invention

This invention relates to perhydro-1,4-thiazepin-5-one and perhydro-1,4-thiazocin-5-one derivatives and related compounds which are useful as angiotensin converting enzyme inhibitors and as antihypertensives. The compounds of this invention are represented by the following formula:

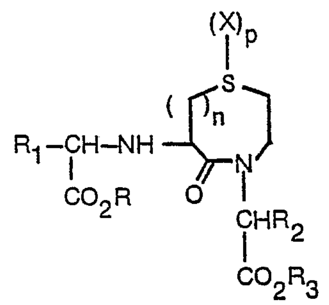

(I)

wherein:
R and $R_3$ are independently hydrogen, loweralkyl, aryl, and aralkyl;
$R_1$ is hydrogen;
alkyl of from 1 to 12 carbon atoms which include branched, cyclic and unsaturated alkyl groups (such as 3-methyl-1-butyl, 2-cyclohexylethyl, 3,3-dimethylallyl;
substituted lower alkyl wherein the substituent can be halo, hydroxy, carboxy, lower alkylthio, lower alkoxy, lower alkoxy carbonyl, lower aralkoxy carbonyl, amino, lower alkylamino, lower dialkylamino, or acylamino;
substituted lower alkyl having the formula $R_A(CH_2)_n$—Q—$(CH_2)_m$ wherein n is 0—2, m is 1—3, $R_A$ is aryl or heteroaryl optionally substituted by amino, lower dialkylamino, lower alkylamino, hydroxy, hydroxy lower alkyl, aminolower alkyl, trihaloloweralkyl, cyano, nitro, sulfonamido, aroyl, lower alkyl, halo, dihalo, and lower alkoxy, and Q is O, S, N—$R_B$, $CONR_C$, $NR_CCO$, CH=CH wherein $R_B$ is hydrogen, loweralkyl, aryl, aralkyl, lower alkanoyl, or aroyl, and $R_C$ is hydrogen or lower alkyl; aryl (such as phenyl, naphthyl or biphenyl); substituted aryl wherein the substituent is lower alkyl, amino lowralkyl, loweralkoxy, aryloxy, aroyl, hydroxy, halo, or dihalo;
aralkyl or heteroaralkyl which include branched lower alkyl groups (such as 2,2-dibenzylethyl);
substituted aralkyl or substituted heteroaralkyl which include branched lower alkyl groups wherein the lower alkyl groups can be substituted by amino, acylamino, or hydroxyl and the aryl and heteroaryl groups can be substituted by halo, dihalo, loweralkyl, hydroxy, loweralkoxy, aryloxy, aroyl, arylthio, amino, amino lower alkyl, lower alkanoylamino, aroylamino, lower dialkylamino, lower alkylamino, hydroxy, hydroxy lower alkyl, trihalo lower alkyl, nitro, cyano, or sulfonamido;
$R_2$ is hydrogen, loweralkyl;
n is 1 or 2;
p is 0 or 1;
X is oxygen; and,
the pharmaceutically acceptable salts thereof.

The lower alkyl groups, except where noted otherwise, represented by any of the variables include straight, branched and unsaturated chain hydrocarbon radicals from one to six carbon atoms, for example, methyl, ethyl, propyl, isopropyl, butyl, isobutyl, t-butyl, pentyl, isopentyl, hexyl or vinyl, allyl, butenyl. The aralkyl and heteroaralkyl groups represented by any of the above variables have from one to six carbon atoms in the alkyl portion thereof and include for example, benzyl, phenethyl, 3,3-diphenylpropyl, 3-indolyl methyl. Halo means chloro, bromo, iodo or fluoro. Aryl where it appears in any of the radicals, except where noted, represents phenyl, naphthyl, or biphenyl. Aroyl includes benzoyl, 1-naphthoyl. Heteroaryl includes, for example, indolyl, thienyl, imidazolyl, furyl, benzimidazolyl, pyridyl, quinolinyl, isoquinolinyl, and benzothienyl. Acylamino refers to lower alkanoylamino and aroylamino groups such as, for example, acetylamino, benzoylamino.

Preferred are those compounds of formula I wherein:
R and $R_3$ are hydrogen, loweralkyl, aryl or aralkyl;
$R_1$ is alkyl of 1—10 carbon atoms which include branched, cyclic and unsaturated alkyl groups;
substituted loweralkyl wherein the substituent can be hydroxy, lower alkylthio, amino lower alkylamino, lower dialkylamino, and acylamino;
substituted lower alkyl having the formula $R_A(CH_2)_n$—Q—$(CH_2)_m$— wherein n is 0—2, m is 1—3, $R_A$ is aryl or heteroaryl optionally substituted by lower alkyl, halo, dihalo, amino, cyano, hydroxy, or lower alkoxy, and Q is O, S, N—$R_B$, $CONR_C$, $NR_CCO$, or CH=CH wherein $R_B$ is hydrogen, lower alkyl, aralkyl, lower alkanoyl, or aroyl and $R_C$ is hydrogen or lower alkyl;
aralkyl or heteroaralkyl which include branched lower alkyl groups;
substituted aralkyl or substituted heteroaralkyl which include branched lower alkyl groups wherein the

lower alkyl substituents can be amino, acylamino, or hydroxy and the aryl and heteroaryl substituents can be lower alkyl, halo, dihalo, amino, cyano, hydroxy, lower alkoxy, amino loweralkyl, or hydroxyloweralkyl;
$R_2$ is hydrogen, lower alkyl;
n is 1 or 2; and,
p is 0.

More preferred are those compounds of Formula I wherein:
R, $R_1$ and $R_2$ are as defined above, n is 1 and p is 0.

Most preferred are those compounds of Formula I wherein:
R and $R_3$ are independently hydrogen, lower alkyl of 1 to 4 carbon atoms, phenyl, or benzyl;
$R_1$ is alkyl of 1—8 carbon atoms which include branched alkyl groups;
substituted lower alkyl wherein the substituent can be amino or loweralkylthio;
substituted lower alkyl having the formula $R_A(CH_2)_n$—Q—$(CH_2)_m$— wherein n is 0, m is 1, $R_A$ is phenyl, and Q is O or S;
aralkyl wherein the aryl is phenyl or naphthyl and the alkyl group contains 1 to 3 carbon atoms, or heteroaralkyl wherein the heteroaryl group is indole, thiophene, imidazole, pyridine, quinoline or isoquinoline and the alkyl group contains 1 to 3 carbon atoms;
substituted aralkyl wherein the aryl is a phenyl group, the alkyl contains 1 to 3 carbon atoms, and the phenyl substituents can be halo, hydroxy, phenoxy, lower alkoxy, amino, or aminomethyl;
$R_2$ is hydrogen, lower alkyl;
n is 1; and,
p is 0.

The preferred, more preferred and most preferred compounds also include the pharmaceutically acceptable salts thereof.

The products of Formula I and the preferred subgroups thereof can be produced by the methods depicted in the following Reaction Scheme wherein R, $R_1$, $R_2$, $R_3$, X, p and n are as defined above.

As will be evident to those skilled in the art and as demonstrated in the Examples, reactive groups not involved in the condensations, such as amino, carboxy, hydroxy, etc., may be protected by methods standard in peptide chemistry prior to the coupling reactions and subsequently deprotected to obtain the desired products.

REACTION SCHEME

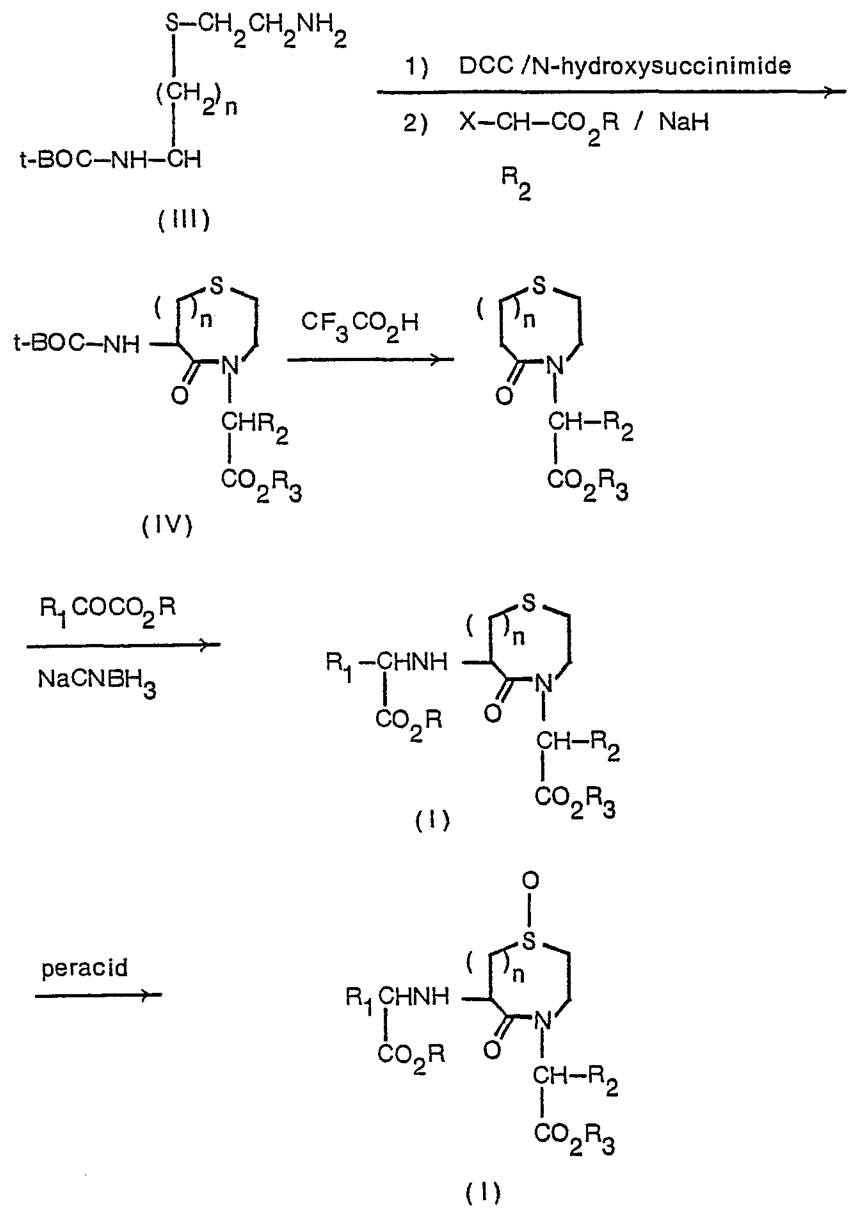


With reference to the Reaction Scheme, the $\alpha$-*t*-BOC derivatives of S-(aminoethyl)cysteine and of S-(aminoethyl)homocysteine (II) are liberated from their corresponding S-(carbonbenzyloxyamino-ethyl) precursors using sodium and liquid ammonia. They are then cyclized to thialactams via carboxyl activating reagents such as dicyclohexylcarbodiimide in the presence of N-hydroxysuccinimide. N-Alkylation of these lactams with $\alpha$-haloesters or $\alpha$-haloacids in the presence of a base such as sodium hydride affords, after chromatography, the lactam intermediate IV. The tertiary butoxycarbonyl group can be removed from compound IV under standard acid conditions to afford the aminothialactam derivative V. Reductive condensation of the latter with $\alpha$-ketoacids or $\alpha$-ketoesters in the presence of sodium cyanoborohydride yields compounds of Formula I. These, in turn, can be converted to sulfoxides by oxidation with a peracid such as m-chloroperbenzoic acid. By a suitable choice of R groups, compounds of Formula I as free diacids, monoesters and diesters are formed. Protecting groups of optional amino functions in the $R_1$ group may be utilized and removed by methods which are standard in peptide chemistry.

The reductive alkylation of $\alpha$-ketoacids or $\alpha$-ketoesters with conpound V generates a new asymmetric center which is preferred in the S configuration. When $R_2$ is lower alkyl, an additional asymmetric center is present in compounds of Formula I. The preferred conformation of this ester is S although the R conformation is also consistent with good activity. The preferred conformation at the

carbon bearing the alkylated amino function in Formula I is that corresponding to a natural L-amino acid. Mixtures of diasteromers of these compounds have activity and may be utilized in the treatment of hypertension. Alternatively, the preferred diasteromer may be isolated by liquid chromotography.

The α-ketocarboxylate derivatives used in the reductive alkylation can include such compounds as, for example ethyl 2-oxo-4-phenylbutyrate, ethyl 4-*p*-chlorophenyl-2-oxobutyrate, ethyl 4-(imidazolyl)-2-oxobutyrate, ethyl 2-oxo-4-(2-thienyl)-butyrate, ethyl 2-oxo-4-(2-naphthyl)-butyrate, ethyl 4-*p*-hydroxyphenyl-2-oxobutyrate, ethyl phenoxypyruvate, ethyl 2-oxo-5-phenylpentanoate, ethyl 4-*p*-methoxyphenyl-2-oxobutyrate, ethyl 5-methyl-2-oxohexanoate, benzyl 2-oxo-6-phthalimido-hexanoate, and the like, and their respective free acids.

The starting materials which are required for the above processes herein described are known in the literature or can be made by known methods from known starting materials.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases e.g., dicyclohexylamine salts, N-methyl-D-glucamine, salts with amino acids like arginine, lysine. Also salts with organic and inorganic acids may be prepared, e.g., HCl, HBr, $H_2SO_4$, $H_3PO_4$, methanesulfonic, toluensulfonic, maleic, fumaric, camphorsulfonic. The non-toxic physiologically acceptable salts are preferred, although other salts are also useful, e.g., in isolating or purifying the product.

The salts may be formed by conventional means as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodepressor substance, bradykinin. Therefore, inhibitors of angiotensin converting enzyme may lower blood-pressure also by protentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., *Biochemistry 16*, 5484 (1977).

The evaluation of converting enzyme inhibitors is guided by *in vitro* enzyme inhibition assays. For example, a useful method is that of Y. Piquilloud, A. Reinharz and M. Roth, *Biochem. Biophys. Acta, 206,* 136 (1970) in which the hydrolysis of carbobenzyloxyphenylalanylhistidinylleucine is measured. *In vivo* evaluations may be made, for example, in normotensive rats challenged with angiotensin I by the technique of J. R. Weeks and J. A. Jones, *Proc. Soc. Exp. Biol. Med., 104,* 646 (1960) or in a high renin rat model such as that of S. Koletsky et al., *Proc. Soc. Exp. Biol. Med. 125,* 96 (1967).

Thus, the compounds of this invention are useful as antihypertensives in treating hypertensive mammals, including humans, and they can be utilized to achieve the reduction of blood pressure by formulating in compositions such as tablets, capsules or elixirs for oral administration or in sterile solutions or suspensions for parenteral administration. The compounds of this invention can be administered to patients in need of such treatment in a dosage range of 2 to 200 mg per patient generally given several times, thus giving a total dose of from 2 to 800 mg per day. The dose will vary depending on severity of disease, weight of patient and other factors which a person skilled in the art will recognize.

Also, the compounds of this invention may be given in combination with other diuretics or antihypertensives. Typically these are combinations whose individual per day dosages range from one-fifth of the minimally recommended clinical dosages to the maximum recommended levels for the entities when they are given singly. To illustrate these combinations, one of the antihypertensives of this invention effective clinically in the range 5—200 milligrams per day can be effectively combined at levels ranging from 1—200 milligrams per day with the following antihypertensives and diuretics in dose ranges per day as indicated:

hydrochlorothiazide (10—100 mg), timolol (5—60 mg), methyldopa (65—2000 mg), the pivaloyloxyethyl ester of methyldopa (30—1000 mg), indacrinone and variable ratios of its enantiomers (25—150 mg), and (+)-4-{3-{[2-(1-hydroxycyclohexyl)ethyl]-4-oxo-2-thiazolidinyl}propyl}benzoic acid (10—100 mg).

In addition, the triple drug combinations of hydrochlorothiazide (10—100 mg) plus amiloride (5—20 mg) plus converting enzyme inhibitor of this invention (1—200 mg) or hydrochlorothiazide (10—100 mg) plus timolol (5—60 mg) plus the converting enzyme inhibitor of this invention (1—200 mg) are effective combinations to control blood pressure in hypertensive patients.

The above dose ranges will be adjusted on a unit basis as necessary to permit divided daily dosage.

Typically, the combinations shown above are formulated into pharmaceutical compositions as discussed below.

About 1 to 100 mg of a compound or mixture of compounds of Formula I or physiologically acceptable salts thereof is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acaria, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil or wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

The following examples are illustrative of the invention and constitute especially preferred embodiments. The preferred diastereomers of these examples are isolated by column chromatography or fractional crystallization. Unless otherwise indicated, all temperatures are given in degrees Celsius and nmr resonances are given in ppm downfield from the TMS resonance.

### Example 1

N-t-Butoxycarbonyl-S-(2-carbobenzyloxyaminoethyl)-L-cysteine

S-(2-Carbobenzyloxyaminoethyl)-L-cysteine (0.6 g) in 6 ml of 2:1 dioxane-water and 2 ml of 1*M* sodium hydroxide solution is treated with di-t-butyl dicarbonate (0.48 g) until reaction is complete. After removal of dioxane, excess reagent is extracted into ether. Upon acidification of the aqueous solution to pH 2 with 4*M* hydrochloric acid, the product separates out as an oil (0.38 g). The NMR spectrum of such a sample shows a broad singlet at 7.6, a singlet at 7.3, a singlet at 5.0, complex multiplets between 2.6 and 3.7, and a singlet at 1.4.

### Example 2

N-t-Butoxycarbonyl-S-(2-aminoethyl)-L-cysteine

N-t-Butoxycarbonyl-S-(2-carbobenzoxyaminoethyl)-L-cysteine (9.9 g) in 500 ml of liquid ammonia is treated with sodium metal until reaction is complete, as indicated by the persistence of a blue color. Ammonium chloride (1 g) is added, followed by ethanol (50 ml). Excess ammonia is let evaporate away. The residue is dissolved in a minimal amount of water, and extracted with ether. The aqueous layer is again taken to dryness, and the residue extracted with ethanol containing 6 ml of ammonium hydroxide. Upon removal of the ethanol the residue obtained is redissolved in 15 ml of water, and passed through 10 ml of Dowex®-50 resin on the ammonium cycle. The resin is washed with 30 ml of water. The product (5.0 g) is obtained upon lyophilization of the eluent. The NMR spectrum (in $D_2O$ and NaOD) of such a sample shows a multiplet at 4.1, complex absorption between 2.6 and 3.0, and a singlet at 1.4.

### Example 3

6-t-Butoxycarbonylamino-perhydro-1,4-thiazepin-5-one

N-t-Butoxycarbonyl-S-(2-aminoethyl)-L-cysteine (5.0 g) and N-hydroxysuccinimide (2.6 g) are dissolved in 500 ml of DMF. With cooling in an ice-bath, a solution of dicyclohexylcarbodiimide (4.7 g) in 10 ml of DMF is added dropwise over a period of 15 minutes. The reaction mixture is kept in a 4° room for 10 days. The solvent is then removed by lyophilization. The crude product is extracted into chloroform. Repeated chromatography on silica gel (20:1 chloroform-methanol) affords the pure product (3.1 g). The NMR spectrum is consistent with the assigned structure. A pure sample is obtained by recrystallization (ether-methanol) and analyzes for $C_{10}H_{18}N_2O_3S$.

### Example 4

6-t-Butoxycarbonylamino-4-N-(carbomethoxymethyl)-perhydro-1,4-thiazepin-5-one

6-t-Butoxycarbonylamino-perhydro-1,4-thiazepin-5-one (0.1 g) and methyl iodoacetate (0.1 g) are dissolved in 1 ml of THF. Sodium hydride (50% suspension in oil) is added in batches. After one hour at room temperature, the reaction is quenched with 2 ml of 10% ammonium chloride solution. The crude product is extracted into methylene chloride, and subsequently chromatographed on silica gel

(40:1 chloroform-methanol) to yield the pure product (0.09 g). The NMR spectrum is consistent with the assigned structure. A sample is obtained by recrystallization from ether-methanol and analyzes for $C_{13}H_{22}N_2O_5S$.

Example 5

6-Amino-4-N-(carbomethoxymethyl)-perhydro-1,4-thiazepin-5-one, Trifluoroacetate Salt

6-t-Butoxycarbonylamino-4-N-(carbomethoxymethyl)-perhydro-1,4-thiazepin-5-one (0.53 g) is dissolved in 2 ml of trifluoroacetic acid. At the end of one hour at room temperature the trifluoroacetic acid is removed under vacuum. The residue is partitioned between water and ether. The aqueous layer is washed three more times with ether, and then lyophilized to yield the product as a white solid (0.52 g).

Example 6

4-N-(Carbomethoxymethyl)-6-(carboethoxy-3-phenylpropylamino)-perhydro-1,4-thiazepin-5-one

6-Amino-4-N-(carbomethoxymethyl)perhydro-1,4-thiazepin-5-one, trifluoroacetate salt (0.166 g), sodium acetate (0.04 g), and ethyl 2-keto-4-phenylbutyrate are dissolved in 1 ml of methanol. After 1/2 hour at room temperature, a solution of sodium cyanoborohydride (0.16 g) in 0.5 ml of methanol is added dropwise over a period of one hour. At the end of seven hours, the reaction mixture is partitioned between chloroform and water. The aqueous phase is adjusted to pH 10 with sodium bicarbonate. The organic layer is separated and washed two more times with water. After chromatography on silica gel (1:1 ethyl acetate-hexanes), the product is obtained as an oil (0.17 g). The NMR spectrum shows a singlet at 7.2, complex absorption between 3.7 and 4.4, a multiplet between 3.1 and 3.4, a multiplet between 3.6 and 3.9, a multipet between 1.9 and 2.2, and a triplet at 1.3.

Example 7

4-N-(Carboxymethyl)-6-[carboxy-3-phenylpropylamino]-perhydro-1,4-thiazepin-5-one

4-[Carbomethoxymethyl]-6-[carboethoxy-3-phenylpropylamino]-perhydro-1,4-thiazepin-5-one (0.1 g) is dissolved in 1.5 ml of methanol and 0.5 ml of 1*M* sodium hydroxide. At the end of 4 hours at room temperature, 2 ml of water is added. The resulting solution is loaded onto a column of Dowex®-50 resin (on the proton cycle). After thorough washing with water, the column is developed with 5% pyridine. The eluent is lyophilized to yield the product as a white solid (0.085 g). The sample analyzes for $C_{17}H_{23}N_2O_5S.1/2\ H_2O$.

Example 8

6-t-Butoxycarbonylamino-4-N-(1-carbomethoxyethyl)-perhydro-1,4-thiazepin-5-one

The above-named compound is prepared from 6-t-butoxycarbonylamino-perhydro-1,4-thiazepin-5-one (0.1 g) and methyl iodopropionate (0.11 g) according to the procedure described in Example 4. The product (0.11 g) is obtained after chromatography. The NMR spectrum is consistent with the assigned structure.

Example 9

6-Amino-4-N-[1-carboxyethyl]perhydro-1,4-thiazepin-5-one

6-t-Butoxycarbonylamino-4-N-[1-carbomethoxyethyl]-perhydro-1,4-thiazepin-5-one (0.11 g) is treated with trifluoroacetic acid and worked up as described in Example 5. The white solid (0.095 g) obtained after lyophilization is dissolved in 2 ml of methanol and 1 ml of 1*M* sodium hydroxide. At the end of four hours at room temperature, the solution is titrated to pH 7 with 1*M* hydrochloric acid, and subsequently loaded onto a column of Dowex®-50 (on the proton cycle). After thorough washing with water, the column is developed with a 5% pyridine solution. The eluent is lyphilized to afford the product as a white powdery solid (0.05 g). The NMR spectrum (in $D_2O$) shows a multiplet from 4.0 to 4.3, a multiplet from 3.1 to 3.4, a doublet at 1.8 and a doublet at 1.7.

Example 10

4-N-(1-carboxyethyl)-6-(1-carboethoxy-3-phenylpropylamino)-perhydro-1,4-thiazepin-5-one

6-Amino-4-N-(1-carboxyethyl)perhydro-1,4-thiazepin-5-one (0.05 g) is suspended in 1 ml of methanol. Ethyl 2-ketone-4-phenylbutyrate (0.24 g) and acetic acid (0.02 g) are added. A 1*M* solution of sodium cyanoborohydride (0.75 ml) is added dropwise over a period of twenty hours. After removal of methanol, the reaction mixture is taken up in water and loaded onto a column of Dowex®-50 (on the proton cycle). After thorough washing with water, the column is developed with 5% pyridine solution. Lyophilization of the eluent afforded the crude product (0.08 g), which is purified by preparative thin layer chromatography on silica gel (9:1 chloroform-methanol containing 5% formic acid). The product is obtained as an oil (0.07 g). The NMR spectrum is consistent with the assigned structure.

Example 11

4-N-(carbomethoxymethyl)-6-(1-carboethoxy-3-phenylpropylamino)-perhydro-1,4-thiazepin-5-one, 1-S-oxide

4-N-(carbomethoxymethyl)-6-(carboethoxy-3-phenylpropylamino)-perhydro-1,4-thiazepin-5-one (0.04 g) is dissolved in 1 ml of methylene chloride, and the solution cooled in an ice-bath. m-Chloroperbenzoic acid (0.024 g) is added. At the end of one hour, thein layer chromatography on silica gel (10:1 chloroform-methanol) shows completion of reaction. The reaction mixtrure is washed with saturated sodium bicarbonate solution and water. Usual work-up of the organic layer yielded the crude product as a yellow glass (0.045 g) which is purified by chromatography on silica gel (9:1 chloroformmethanol) to afford a yellow glass (0.04 g). The NMR spectrum is consistent with the assigned structure.

Example 12

4-N-(Carboxymethyl)-6-(1-carboxy-3-phenylpropylamino)-perhydro-1,4-thiazepin-5-one, 1-S-oxide

4-N-(carbomethoxymethyl)-6-(carboethoxy-3-phenylpropylamino) - perhydro - 1,4 - thiazepin - 5-one, 1-S-oxide (0.05 g) is dissolved in 1 ml of methanol and 0.3 ml of 1*M* sodium hydroxide. After four hours at room temperature, 5 ml of water is added and the solution titrated to pH 7 with 1*M* hydrochloric acid. The solution is loaded onto a column of Dowex®-50 (on the proton cycle). After thorough washing with water, the column is developed with 5% pyridine solution. Lyophilization of the eluent afforded the product as a white solid (0.02 g). The NMR spectrum is consistent with the assigned structure.

Example 13

N-t-Butoxycarbonyl-D,L-homocysteine thiolactone

D,L-Homocysteine thiolactone, hydrochloride (0.153 g) and triethylamine (0.15 ml) are dissolved in 2 ml of chloroform. Di-t-butyldicarbonate (0.22 g) is added. After 1/2 hour, the reaction mixture is washed with dilute hydrochloric acid, and saturated sodium chloride solution. Usual work-up of the organic layer afforded the product as a white crystalline solid (0.2 g). The NMR spectrum shows a broad doublet at 5.3, a quintet at 4.3, a multiplet at from 3.2 to 3.5, a complex absorption between 2.0 and 3.0, and a singlet at 1.5.

Example 14

N-t-Butoxycarbonyl-S-[2-carbobenzoxyaminoethyl]-D,L-homocysteine

N-t-Butoxycarbonyl-D,L-homocysteine thiolactone (0.22 g) is dissolved in 2 ml each of dioxane and 1*M* sodium hydroxide. N-carbobenzoxycarbonyl-2-iodoethylamine (0.31 g) is added. After standing over-night at room temperature, the solution is concentrated to remove most of the dioxane. The aqueous solution is acidified with dilute hydrochloric acid, and extracted with methylene chloride. The organic layer is washed with saturated sodium chloride solution and worked up as usual to yield the product as an oil (0.36 g). The NMR spectrum shows a singlet at 7.3, a broad singlet at 6.9, a singlet at 5.1, a multiplet between 4.1 and 4.4, a multiplet between 3.1 and 3.5, a multiplet between 2.5 and 2.8, a multiplet between 1.8 and 2.2, and a singlet at 1.5.

Example 15

N-t-Butoxycarbonyl-S-(2-aminoethyl)-D,L-homocysteine

N-t-Butoxycarbonyl-S-(2-carbobenzyloxycarbonyl aminoethyl)-D,L-homocysteine (1.0 g) is treated with sodium in liquid ammonia according to the procedure described in Example 2. After the ammonia has evaporated off, the residue is extracted with ethanol. Inorganic salts (0.4 g) is removed by filtration. After removal of ethanol, the residue is taken up in 5 ml of water. The aqueous solution is extracted with ether, and then acidified to pH 4 with 4*M* hydrochloric acid. The solution is concentrated to a volume of 2 ml. Excess ether is added. The product crystallizes out as a white solid (0.32 g). The NMR spectrum (in MeOH-$d_4$ and NaOD) shows a triplet at 3.7, complex absorption between 2.1 and 2.6, a multiplet between 1.5 and 1.9, and a singlet at 1.2.

Example 16

6-t-Butoxycarbonylamino-perhydro-1,4-thiazocin-5-one

N-t-Butoxycarbonyl-S-(2-aminoethyl)-D,L-homocysteine (0.1 g) and hydroxysuccinimide (0.044 g) are dissolved in 4 ml of DMF. Dicyclohexylcarbodiimide (0.086 g) in 1 ml of DMF is added dropwise. After 4 days at room temperature, the reaction mixture is filtered, and then lyophilized. The residue is taken up in methylene chloride. Again insoluble white solid is removed by filtration. The crude product is chromatographed on silica gel (20:1 chloroformmethanol) to afford the product as a white solid (0.02 g). The NMR spectrum (in meOH-$d_4$) shows a multiplet between 4.4 and 4.8, a multiplet between 3.2 and 3.4, complex absorptions between 2.2 and 2.7, and between 1.5 and 1.9, and a singlet at 1.2.

Example 17

6-t-Butoxycarbonylamino-4-N-(carbo-t-butoxymethyl)perhydro-1,4-thiazocin-5-one

The above-named compound is prepared from 6-t-butoxycarbonylamino-perhydro-1,4-thiazocin-5-one (0.1 g) and t-butyl iodoacetate (0.2 g) according to the procedure described in Example 4. The product (0.05 g) is obtained after chromatography. The NMR spectrum is consistent with the assigned structure. A recrystallized sample (ether) analyzes for $C_{17}H_{30}N_2O_5S$.

Example 18

4-N-(Carboxymethyl)-6-(1-carboethoxy-3-phenylpropylamino)-perhydro-1,4-thiazocin-5-one

Removal of the t-butyl ester and t-butoxycarbonyl groups of 6-t-butoxycarbonylamino-4-N-(carbo-t-butoxymethyl)-perhydro-1,4-thiazocin-5-one with trifluoroacetic acid as in Example 5 followed by reductive condensation of this product with ethyl 2-keto-4-phenylbutyrate using sodium cyanoborohydride as in Example 6 affords, after chromatography, 4-N-(carboxymethyl)-6-(1-carboethoxy-3-phenylpropylamino)-perhydro-1,4-thiazocin-5-one.

Example 19

By methods illustrated in the above examples, the keto acids and esters listed in Table I below can be reductively condensed using sodium cyanoborohydride with 6-amino-4-(1-carboxyethyl)perhydro-1,4-thiazepin-5-one, 6-amino-4-carboxymethylperhydro-1,4-thiazepin-5-one or with their ethyl esters which can be prepared by standard Fisher esterification. Thusly obtained additional products of Formula I, after removal of protecting groups if any, are listed hereinbelow in Table II.

TABLE I

KETO ACIDS AND KETO ESTERS OF THE FORMULA:
$R_1COCO_2R$

(a) 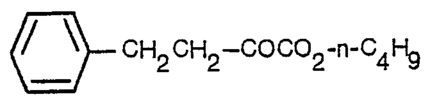

(b) 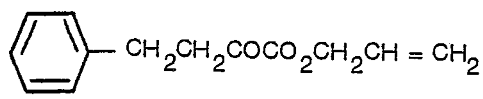

(c) 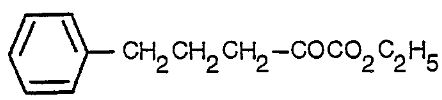

(d) 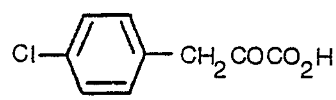

(e) 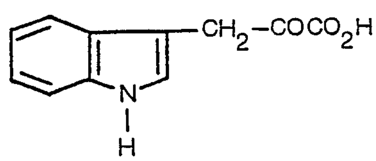

(f) 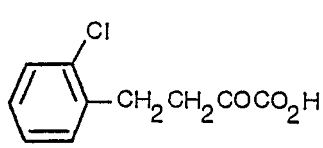

(g) 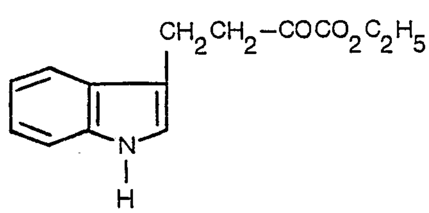

(h) 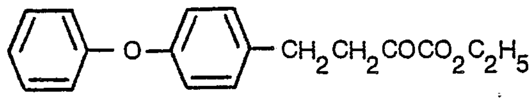

(i) 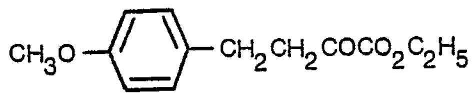

(j) 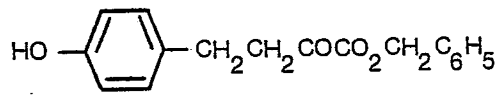

(k) 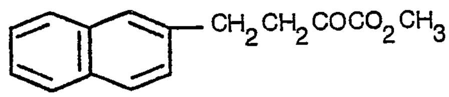

(l) 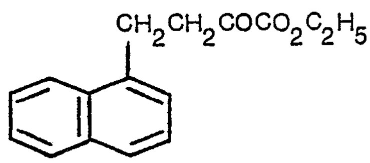

(m) 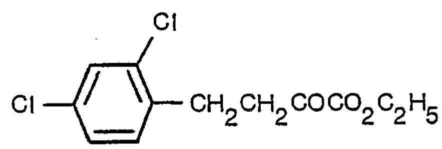

(n) 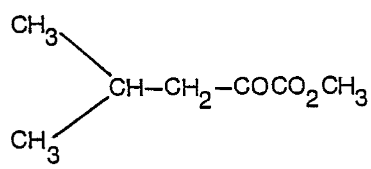

(o) $CH_3CH_2CH_2CH_2-COCO_2C_2H_5$

(p) 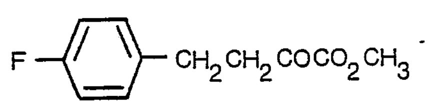

(q) $NH_2-(CH_2)_5-COCO_2H$ (1)

(r) 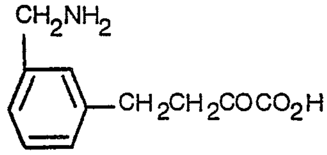 (1)

(s) 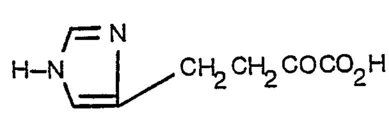 (2)

(t) 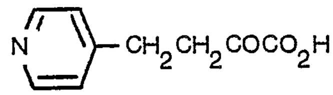

(u) 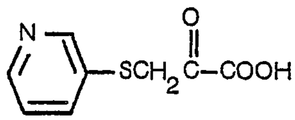

(v) $HOCH_2CH_2 - COCO_2C_2H_5$ (3)

(w) 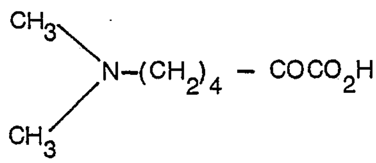

(x) 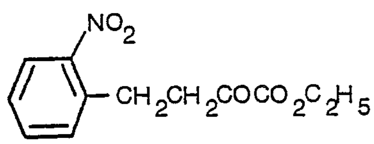 (4)

(k) 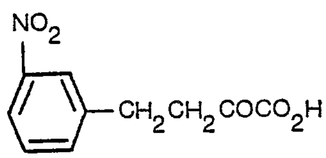 (4)

(z) 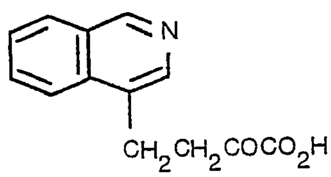

(aa) 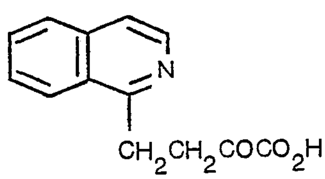

(bb) 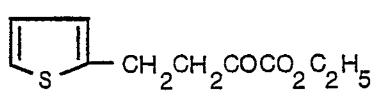

(cc) 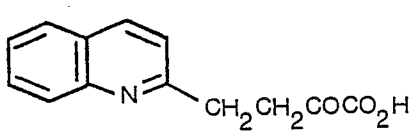

(dd) 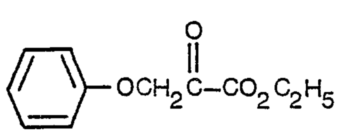

(ee) 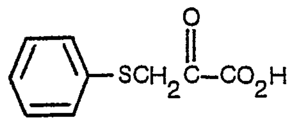

(ff) 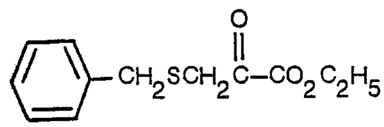

(1) Protected as the N-phthaloyl derivative.
(2) 2-Imidazole NH protected as the N-benzyl derivative.
(3) Protected as the O-benzyl derivative.
(4) Precursor to m-amino derivative by $H_2$/Pd.

TABLE II

Additional Products of Formula I:

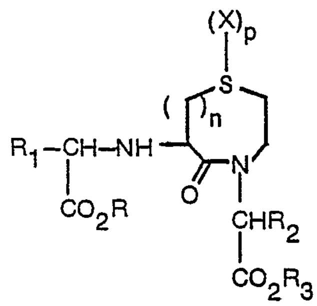

(n=1; p=0)

| | R | $R_1$ | $R_2$ | $R_3$ |
|---|---|---|---|---|
| (1) | n-$C_4H_9$– | 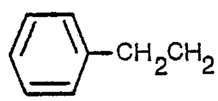 | H | H |
| (2) | $CH_2$=CH–$CH_2$– | 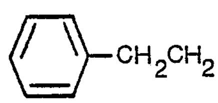 | H | $C_2H_5$ |
| (3) | $C_2H_5$– | 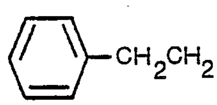 | H | $C_2H_5$ |
| (4) | $C_2H_5$– | 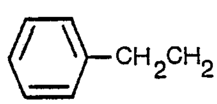 | H | $CH_2$=CH–$CH_2$ (5) |
| (5) | 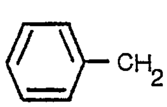 | 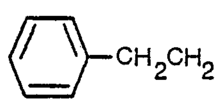 | H | H |
| (6) | $CH_3$– | 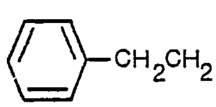 | H | n-$C_4H_9$– (5) |

TABLE II (Continued)

| R | $R_1$ | $R_2$ | $R_3$ |
|---|---|---|---|
| (7) H– | 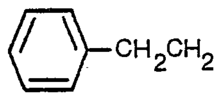 | H | 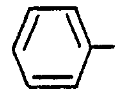 (5) |
| (8) $CH_3$– | 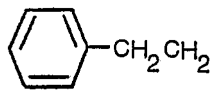 | H | n-$C_4H_9$ (5) |
| (9) $CH_3$– | 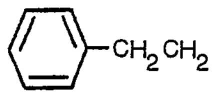 | H | $C_2H_5$– |
| (10) $CH_3$– | 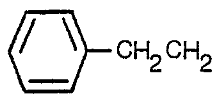 | H | 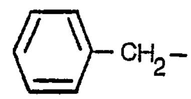 |
| (11) H | 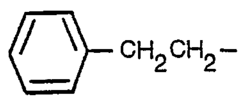 | H | 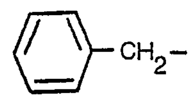 |
| (12) $C_2H_5$– | 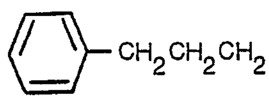 | H | H |
| (13) H | 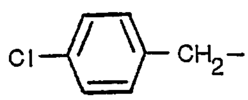 | H | H |
| (14) H | 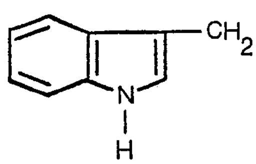 | H | H |
| (15) H | 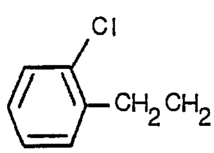 | H | H |
| (16) $C_2H_5$– | 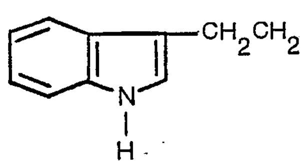 | H | $CH_3$ |
| (17) $C_2H_5$– | 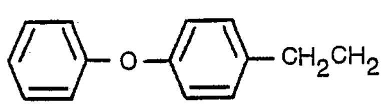 | H | H |

TABLE II (Continued)

| | R | $R_1$ | $R_2$ | $R_3$ |
|---|---|---|---|---|
| (18) | $C_2H_5-$ | 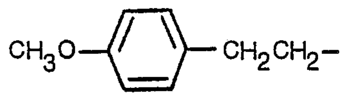 | H | n-$C_4H_9$ |
| (19) | 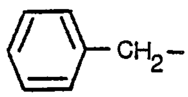 | 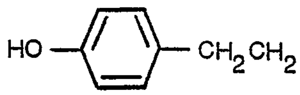 | H | H |
| (20) | $CH_3-$ | 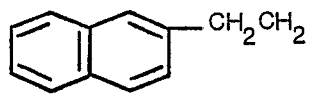 | H | $C_2H_5$ |
| (21) | $C_2H_5$ | 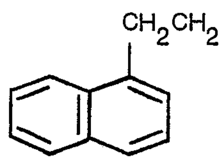 | H | 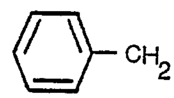 |
| (22) | $C_2H_5-$ | $CH_3S-CH_2CH_2-$ | H | H |
| (23) | $CH_3$ | $(CH_3)_2-CH-CH_2-$ | H | n-$C_4H_9$ |
| (24) | $C_2H_5-$ | $CH_3CH_2CH_2CH_2$ | H | $C_2H_5$ |
| (25) | $CH_3-$ | 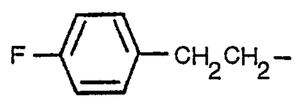 | H | 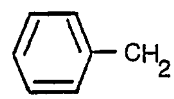 |
| (26) | H | $NH_2(CH_2)_5-$ | H | H |
| (27) | H | 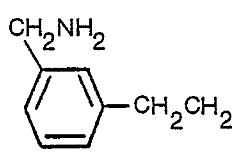 | H | H |
| (28) | H | 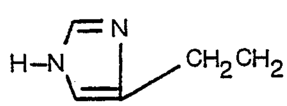 | H | H |
| (29) | H | 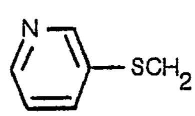 | H | H |
| (30) | $C_2H_5$ | $HOCH_2CH_2-$ | H | $C_2H_5$ |

TABLE II (Continued)

| | R | $R_1$ | $R_2$ | $R_3$ |
|---|---|---|---|---|
| (31) | H | 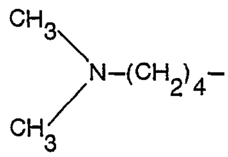 | H | H |
| (32) | $C_2H_5$ | 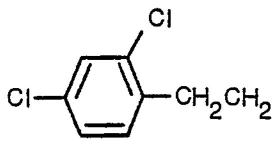 | S | 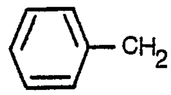 |
| (33) | H | 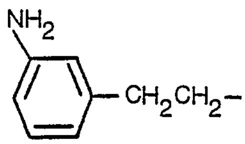 | H | 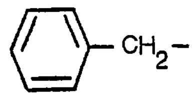 |
| (34) | H | 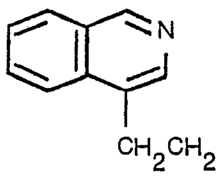 | H | H |
| (35) | H | 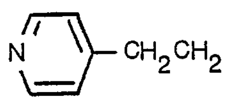 | H | H |
| (36) | $C_2H_5$ | 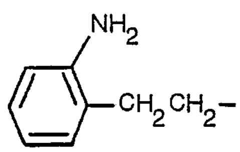 | H | H |
| (37) | $C_2H_5$ | 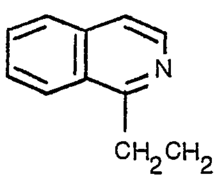 | H | $C_2H_5$ |
| (38) | $C_2H_5$ | 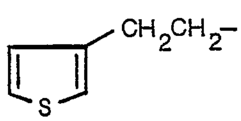 | S | $CH_2=CH-CH_2$ |
| (39) | H | 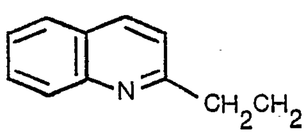 | H | H |

TABLE II (Continued)

| | R | $R_1$ | $R_2$ | $R_3$ |
|---|---|---|---|---|
| (40) | $C_2H_5$ | 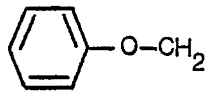 | H | H |
| (41) | H | 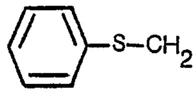 | H | H |
| (42) | $C_2H_5$ | 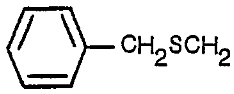 | H | H |
| (43) | $n\text{-}C_4H_9-$ | 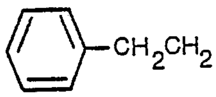 | $-CH_3$ | H |
| (44) | $CH_2=CH-CH_2-$ | 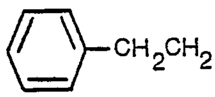 | $-CH_3$ | $C_2H_5$ |
| (45) | $C_2H_5-$ | 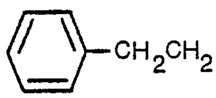 | $-CH_3$ | $C_2H_5$ |
| (46) | $C_2H_5-$ | 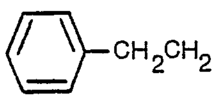 | $-CH_3$ | $CH_2=CH-CH_2$ (5) |
| (47) | 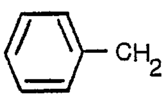 | 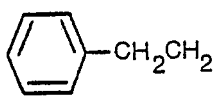 | $-CH_3$ | H |
| (48) | $CH_3-$ | 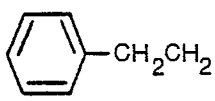 | $-CH_3$ | $n\text{-}C_4H_9-$ (5) |
| (49) | H– | 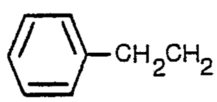 | $-CH_3$ | 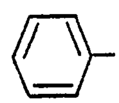 (5) |
| (50) | $CH_3-$ | 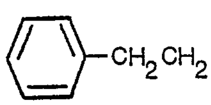 | $-CH_3$ | $n\text{-}C_4H_9$ (5) |
| (51) | $CH_3-$ | 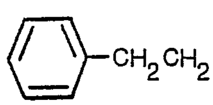 | $-CH_3$ | $C_2H_5-$ |

TABLE II (Continued)

| R | $R_1$ | $R_2$ | $R_3$ |
|---|---|---|---|
| (52) $CH_3-$ | 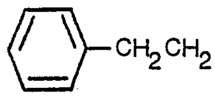 | $-CH_3$ | 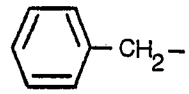 |
| (53) H | 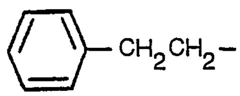 | $-CH_3$ | 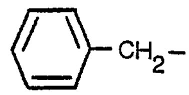 |
| (54) $C_2H_5-$ | 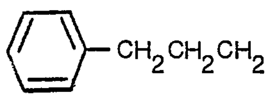 | $-CH_3$ | H |
| (55) H | 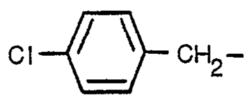 | $-CH_3$ | H |
| (56) H | 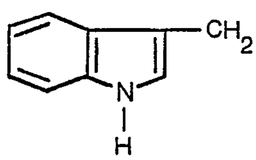 | $-CH_3$ | H |
| (57) H | 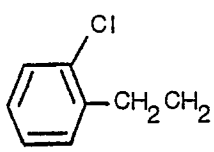 | $-CH_3$ | H |
| (58) $C_2H_5-$ | 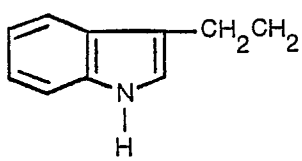 | $-CH_3$ | $CH_3$ |
| (59) $C_2H_5-$ | 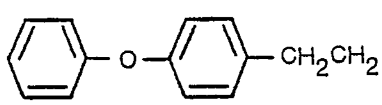 | $CH_3$ | H |
| (60) $C_2H_5-$ | 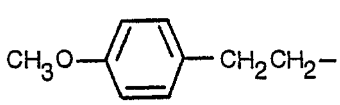 | $CH_3$ | $n-C_4H_9$ |
| (61) 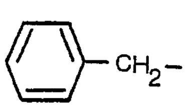 | 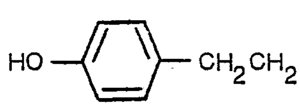 | $CH_3$ | H |
| (62) $CH_3-$ | 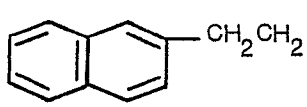 | $CH_3$ | $C_2H_5$ |

TABLE II (Continued)

| | R | $R_1$ | $R_2$ | $R_3$ |
|---|---|---|---|---|
| (63) | $C_2H_5$ | 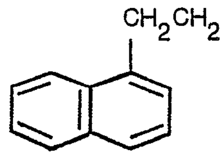 | $CH_3$ | 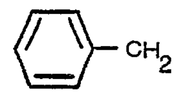 |
| (64) | $C_2H_5-$ | $CH_3S-CH_2CH_2-$ | $CH_3$ | H |
| (65) | $CH_3$ | $(CH_3)_2-CH-CH_2-$ | $CH_3$ | $n\text{-}C_4H_9$ |
| (66) | $C_2H_5-$ | $CH_3CH_2CH_2CH_2$ | $CH_3$ | $C_2H_5$ |
| (67) | $CH_3-$ | 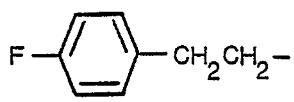 | $CH_3$ | 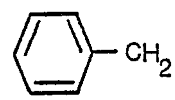 |
| (68) | H | $NH_2(CH_2)_5-$ | $CH_3$ | H |
| (69) | H | 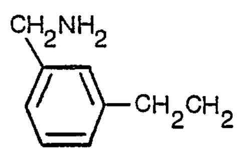 | $CH_3$ | H |
| (70) | H | 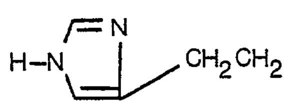 | $CH_3$ | H |
| (71) | H | 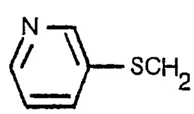 | $CH_3$ | H |
| (72) | $C_2H_5$ | $HOCH_2CH_2-$ | $CH_3$ | $C_2H_5$ |
| (73) | H | 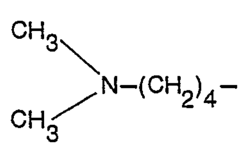 | $CH_3$ | H |
| (74) | $C_2H_5$ | 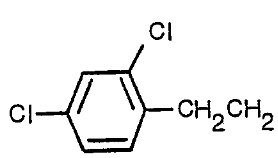 | $CH_3$ | 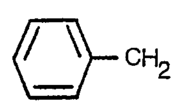 |

TABLE II (Continued)

| R | $R_1$ | $R_2$ | $R_3$ |
|---|---|---|---|
| (75) H | 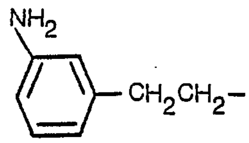 | $CH_3$ | 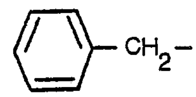 |
| (76) H | 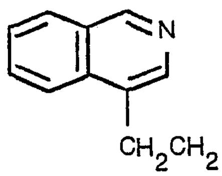 | $CH_3$ | H |
| (77) H | 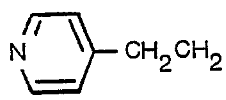 | $CH_3$ | H |
| (78) $C_2H_5$ | 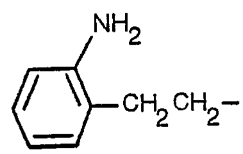 | $CH_3$ | H |
| (79) $C_2H_5$ | 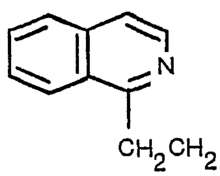 | $CH_3$ | $C_2H_5$ |
| (80) $C_2H_5$ | 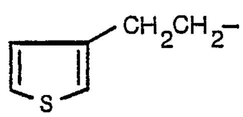 | $CH_3$ | $CH_2=CH-CH_2$ |
| (81) H | 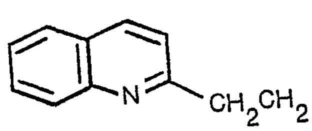 | $CH_3$ | H |
| (82) $C_2H_5$ | 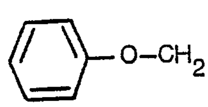 | $CH_3$ | H |
| (83) H | 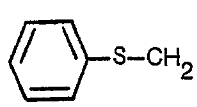 | $CH_3$ | H |
| (84) $C_2H_5$ | 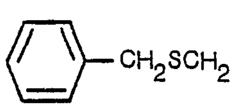 | $CH_3$ | H |

(5) The required $R_2$ ester can be prepared by first protecting VIII as its *t*-BOC derivative and then reacting it with DCC and the desired alcohol or phenol in the presence of 4-dimethylamino pyridine. In some examples, the $R_2$ ester can also be introduced by reaction of protected VIII with cesium carbonate and the appropriate alkyl halide in DMF.

**Claims for the Contracting States: BE CH DE FR GB IT LI LU NL SE**

1. A compound of the formula:

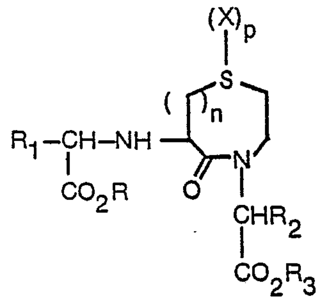

(I)

wherein:
R and $R_3$ are independently hydrogen, loweralkyl, aryl, and aralkyl;
$R_1$ is hydrogen;
alkyl of from 1 to 12 carbon atoms which include branched, cyclic and unsaturated alkyl groups;
substituted lower alkyl wherein the substituent can be halo, hydroxy, carboxy, lower alkylthio, lower alkoxy, lower alkoxy carbonyl, lower aralkoxy carbonyl, amino, lower alkylamino, lower dialkylamino, or acylamino;
substituted lower alkyl having the formula $R_A(CH_2)_n$—Q—$(CH_2)_m$ wherein n is 0—2, m is 1—3, $R_A$ is aryl or heteroaryl optionally substituted by amino, lower dialkylamino, lower alkylamino, hydroxy, hydroxy lower alkyl, aminolower alkyl, trihaloloweralkyl, cyano, nitro, sulfonamido, aroyl, lower alkyl, halo, dihalo, and lower alkoxy, and Q is O, S, N—$R_B$, $CONR_C$, $NR_CCO$, CH=CH wherein $R_B$ is hydrogen, loweralkyl, aryl, aralkyl, lower alkanoyl, or aroyl, and $R_C$ is hydrogen or lower alkyl;
aryl;
substituted aryl wherein the substituent is lower alkyl, amino lowralkyl, loweralkoxy, aryloxy, aroyl, hydroxy, halo, or dihalo;
aralkyl or heteroaralkyl which include branched lower alkyl groups;
substituted aralkyl or substituted heteroaralkyl which include branched lower alkyl groups wherein the lower alkyl groups can be substituted by amino, acylamino, or hydroxyl and the aryl and heteroaryl groups can be substituted by halo, dihalo, loweralkyl, hydroxy, loweralkoxy, aryloxy, aroyl, arylthio, amino, amino lower alkyl, lower alkanoylamino, aroylamino, lower dialkylamino, lower alkylamino, hydroxy, hydroxy lower alkyl, trihalo lower alkyl, nitro, cyano, or sulfonamido;
$R_2$ is hydrogen, loweralkyl;
n is 1 or 2;
p is 0 or 1;
X is oxygen; and,
the pharmaceutically acceptable salts thereof.

2. A compound of Claim 1, wherein
$R_1$ is alkyl of 1—10 carbon atoms which include branched, cyclic and unsaturated alkyl groups;
substituted loweralkyl wherein the substituent can be hydroxy, lower alkylthio, amino lower alkylamino, lower dialkylamino, and acylamino;
substituted lower alkyl having the formula $R_A(CH_2)_n$—Q—$(CH_2)_m$— wherein n is 0—2, m is 1—3, $R_A$ is aryl or heteroaryl optionally substituted by alkyl, halo, dihalo, amino, cyano, hydroxy, or lower alkoxy, and Q is O, S, N—$R_B$, $CONR_C$, $NR_CCO$, or CH=CH wherein $R_B$ is hydrogen, lower alkyl, aralkyl, lower alkanoyl, or aroyl and $R_C$ is hydrogen or lower alkyl;
aralkyl or heteroaralkyl which include branched lower alkyl groups;

3. The compound of Claim 1 which is a member of the group:
4-N-(1(S)-carboxyethyl)-6(R)-(1(S)-carboethoxy-3-phenylpropylamino)perhydro-1,4-thiazepin-5-one;
4-N-(1(S)-carboxyethyl)-6(R)-(1(S)-carboxy-3-phenylpropylamino)perhydro-1,4-thiazepin-5-one;
4-N-carboxymethyl)-6(R)-(1(S)-carboethoxy-3-phenylpropylamino)perhydro-1,4-thiazepin-5-one; and,
4-N-carboxymethyl-6(R)-(1(S)-carboxy-3-phenylpropylamino)perhydro-1,4-thiazepin-5-one.

4. A pharmaceutical composition useful in the treatment of hypertension which comprises a pharmaceutically acceptable carrier and a pharmaceutically effective amount of a compound of the formula:

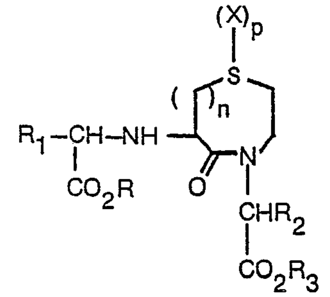

(I)

wherein
R and $R_3$ are independently hydrogen, loweralkyl, aryl, and aralkyl;
$R_1$ is hydrogen;
alkyl of from 1 to 12 carbon atoms which include branched, cyclic and unsaturated alkyl groups;
substituted lower alkyl wherein the substituent can be halo, hydroxy, carboxy, lower alkylthio, lower alkoxy, lower alkoxy carbonyl, lower aralkoxy carbonyl, amino, lower alkylamino, lower dialkylamino, or acylamino;
substituted lower alkyl having the formula $R_A(CH_2)_n—Q—(CH_2)_m$ wherein n is 0—2, m is 1—3, $R_A$ is aryl or heteroaryl optionally substituted by amino, lower dialkylamino, lower alkylamino, hydroxy, hydroxy lower alkyl, aminolower alkyl, trihaloloweralkyl, cyano, nitro, sulfonamido, aroyl, lower alkyl, halo, dihalo, and lower alkoxy, and Q is O, S, $N—R_B$, $CONR_C$, $NR_CCO$, CH=CH wherein $R_B$ is hydrogen, loweralkyl, aryl, aralkyl, lower alkanoyl, or aroyl, and $R_C$ is hydrogen or lower alkyl;
aryl;
substituted aryl wherein the substituent is lower alkyl, amino loweralkyl, loweralkoxy, aryloxy, aroyl, hydroxy, halo, or dihalo;
aralkyl or heteroaralkyl which include branched lower alkyl groups;
substituted aralkyl or substituted heteroaralkyl which include branched lower alkyl groups wherein the lower alkyl groups can be substituted by amino, acylamino, or hydroxyl and the aryl and heteroaryl groups can be substituted by halo, dihalo, loweralkyl, hydroxy, loweralkoxy, aryloxy, aroyl, arylthio, amino, amino lower alkyl, lower alkanoylamino, aroylamino, lower dialkylamino, lower alkylamino, hydroxy, hydroxy loweralkyl, trihalo loweralkyl, nitro, cyano, or sulfonamido;
$R_2$ is hydrogen, loweralkyl;
n is 1 or 2;
p is 0 or 1;
X is oxygen; and,
the pharmaceutically acceptable salts thereof.

5. The composition of Claim 4 wherein said compound is a member of the group:
4-N-(1(S)-carboxyethyl)-6(R)-(1(S)-carboethoxy-3-phenyl-propylamino)perhydro-1,4-thiazepin-5-one;
4-N-(1(S)-carboxyethyl)-6(R)-(1(S)-carboxy-3-phenylpropylamino)perhydro-1,4-thiazepin-5-one;
4-N-carboxyethyl)-6(R)-(1(S)-carboethoxy-3-phenylpropylamino)perhydro-1,4-thiazepin-5-one; and,
4-N-carboxyethyl)-6(R)-(1(S)-carboxy-3-phenylpropylamino)perhydro-1,4-thiazepin-5-one.

6. The composition of Claim 4 which includes an antihypertensive and/or diuretic compound selected from the group consisting of hydrochlorothiazide, methyldopa, timolol, the pivaloyloxyethyl ester of methyldopa, indacrinone and variable ratios of its enantiomers, (+)-4-{3-{[2-(1-hydroxycyclohexyl)ethyl]-4-oxo-2-thiazolidinyl}propyl}benzoic acid, as well as admixtures and combinations thereof.

7. A process for preparing a compound having the formula:

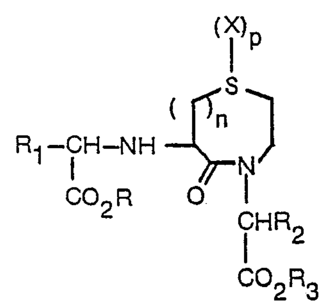

wherein R, $R_1$, $R_2$, $R_3$, X, n and p are as defined in Claim 1, which process comprises: reductively

condensing an $\alpha$-ketoacid or $\alpha$-ketoester having the formula $R_1$ CO—$CO_2R$ wherein R and $R_1$ are as defined in Claim 1 with an $\alpha$-aminothialactam of the formula:

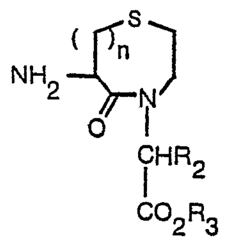

wherein $R_2$, $R_3$ and n are as defined in Claim 1, to afford, after optionally reacting with a peracid followed by removal of protecting groups, if necessary, the desired product and, if desired, isolating the biologically more active isomer by chromatography or fractional crystallization and, if further desired, preparing a salt thereof by conventional means.

**Claims for the contracting state AT**

1. A process for preparing a compound having the formula:

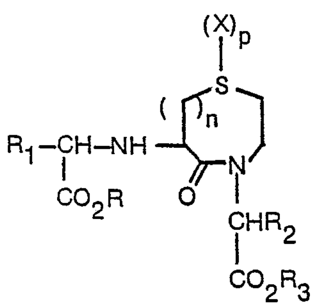

(I)

wherein
R and $R_3$ are independently hydrogen, loweralkyl, aryl, and aralkyl;
$R_1$ is hydrogen;
alkyl of from 1 to 12 carbon atoms which include branched, cyclic and unsaturated alkyl groups;
substituted lower alkyl wherein the substituent can be halo, hydroxy, carboxy, lower alkylthio, lower alkoxy, lower alkoxy carbonyl, lower aralkoxy carbonyl, amino, lower alkylamino, lower dialkylamino, or acylamino;
substituted lower alkyl having the formula $R_A(CH_2)_n$—Q—$(CH_2)_m$ wherein n is 0—2, m is 1—3, $R_A$ is aryl or heteroaryl optionally substituted by amino, lower dialkylamino, lower alkylamino, hydroxy, hydroxy loweralkyl, aminoloweralkyl, trihaloloweralkyl, cyano, nitro, sulfonamido, aroyl, lower alkyl, halo, dihalo, and lower alkoxy, and Q is O, S, N—$R_B$, $CONR_C$, $NR_CCO$, CH=CH wherein $R_B$ is hydrogen, loweralkyl, aryl, aralkyl, lower alkanoyl, or aroyl, and $R_C$ is hydrogen or lower alkyl;
aryl;
substituted aryl wherein the substituent is lower alkyl, amino lowralkyl, loweralkoxy, aryloxy, aroyl, hydroxy, halo, or dihalo;
aralkyl or heteroaralkyl which include branched lower alkyl groups;
substituted aralkyl or substituted heteroaralkyl which include branched lower alkyl groups wherein the lower alkyl groups can be substituted by amino, acylamino, or hydroxyl and the aryl and heteroaryl groups can be substituted by halo, dihalo, loweralkyl, hydroxy, loweralkoxy, aryloxy, aroyl, arylthio, amino, amino lower alkyl, lower alkanoylamino, aroylamino, lower dialkylamino, lower alkylamino, hydroxy, hydroxy loweralkyl, trihalo loweralkyl, nitro, cyano, or sulfonamido;
$R_2$ is hydrogen, loweralkyl;
n is 1 or 2;
p is 0 or 1;
X is oxygen; and,
the pharmaceutically acceptable salts thereof, which process comprises: reductively condensing an $\alpha$-

ketoacid or α-ketoester having the formula $R_1CO—CO_2R$ wherein R and $R_1$ are as defined above with an α-aminothialactam of the formula:

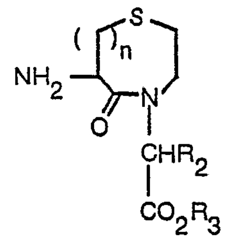

wherein $R_2$, $R_3$ and n are as defined above, to afford, after optionally reacting with a peracid followed by removal of protecting groups, if necessary, the desired product and, if desired, isolating the biologically more active isomer by chromatography or fractional crystallization and, if further desired, preparing a salt thereof by conventional means.

2. The process of Claims 1 for preparing a compound according to Claim 1 wherein
$R_1$ is alkyl of 1—10 carbon atoms which include branched, cyclic and unsaturated alkyl groups;
substituted loweralkyl wherein the substituent can be hydroxy, lower alkylthio, amino, lower alkylamino, lower dialkylamino, and acylamino;
substituted lower alkyl having the formula $R_A(CH_2)_n—Q—(CH_2)_m—$ wherein n is 0—2, m is 1—3, $R_A$ is aryl or heteroaryl optionally substituted by alkyl, halo, dihalo, amino, cyano, hydroxy, or lower alkoxy, and Q is O, S, $N—R_B$, $CONR_C$, $NR_CCO$, or CH=CH wherein $R_B$ is hydrogen, lower alkyl, aralkyl, lower alkanoyl, or aroyl and $R_C$ is hydrogen or lower alkyl;
aralkyl or heteroaralkyl which include branched lower alkyl groups;

3. The process of Claim 1 for preparing a compound according to Claim 1 which is a member of the group:
4-N-(1(S)-carboxyethyl)-6(R)-(1(S)-carboethoxy-3-phenylpropylamino)perhydro-1,4-thiazepin-5-one;
4-N-(1(S)-carboxyethyl)-6(R)-(1(S)-carboxy-3-phenylpropylamino)perhydro-1,4-thiazepin-5-one;
4-N-carboxymethyl)-6(R)-(1(S)-carboethoxy-3-phenylpropylamino)perhydro-1,4-thiazepin-5-one; and,
4-N-carboxymethyl)-6(R)-(1(S)-carboxy-3-phenylpropylamino)perhydro-1,4-thiazepin-5-one.

**Patentansprüche für die Vertragsstaaten: BE CH DE FR GB IT LI LU NL SE**

1. Eine Verbindung der Formel:

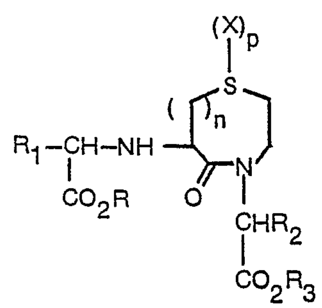

(I)

worin
R und $R_3$ unabhängig voneinander Wasserstoff, Niedrigalkyl, Aryl und Aralkyl sind;
$R_1$ Wasserstoff; Alkyl mit 1 bis 12 Kohlenstoffatomen, welches verzweigte, cyclische und ungesättigte Alkylgruppen umfaßt; substituiertes Niedrigalkyl, worin der Substituent Halogen, Hydroxy, Carboxy, Niedrigalkylthio, Niedrigalkoxy, Niedrigalkoxycarbonyl, Niedrigaralkoxycarbonyl, Amino, Niedrigalkylamino, Niedrigdialkylamino oder Acylamino sein kann; substituiertes Niedrigalkyl mit der Formel $R_A(CH_2)_n—Q—(CH_2)_m$, worin n 0—2 ist, m 1—3 ist, $R_A$ Aryl oder Heteroaryl ist, das gegebenenfalls durch Amino, Niedrigdialkylamino, Niedrigalkylamino, Hydroxy, Hydroxyniedrigalkyl, Aminoniedrigalkyl, Trihalogenniedrigalkyl, Cyano, Nitro, Sulfonamido, Aroyl, Niedrigalkyl, Halogen, Dihalogen und Niedrigalkoxy substituiert ist, und Q O, S, $N—R_B$, $CONR_C$, $NR_CCO$, CH=CH ist, worin $R_B$ Wasserstoff, Niedrigalkyl, Aryl, Aralkyl, Niedrigalkanoyl oder Aroyl ist, $R_C$ Wasserstoff oder Niedrigalkyl ist; Aryl, substituiertes Aryl, worin der Substituent Niedrigalkyl, Aminoniedrigalkyl, Niedrigalkoxy, Aryloxy, Aroyl, Hydroxy, Halogen oder Dihalogen ist; Aralkyl oder Heteroaralkyl, welches verzweigte niedrige Alkylgruppen umfaßt; substituiertes Aralkyl oder substituiertes Heteroaralkyl, welches verzweitge niedrige Alkylgruppen unfaßt, worin die niedrigen Alkylgruppen durch Amino, Acylamino oder Hydroxyl substituiert sein können, und die Aryl- und Heteroarylgruppen durch Halogen, Dihalogen, Niedrigalkyl, Hydroxy,

Niedrigalkoxy, Aryloxy, Aroyl, Arylthio, Amino, Aminoniedrigalkyl, Niedrigalkanoylamino, Aroylamino, Niedrigdialkylamino, Niedrigalkylamino, Hydroxy, Hydroxyniedrigalkyl, Trihalogenniedrigalkyl, Nitro, Cyano oder Sulfonamido substituiert sein können; bedeutet;

$R_2$ Wasserstoff, Niedrigalkyl ist;

n 1 oder 2 ist;

p 0 oder 1 ist;

X Sauerstoff ist; und

die pharmazeutisch annehmbaren Salze davon.

2. Eine Verbindung des Anspruchs 1, worin

$R_1$ Alkyl mit 1 bis 10 Kohlenstoffatomen, welches verzweigte, cyclische und ungesättigte Alkylgruppen umfaßt; substituiertes Niedrigalkyl, worin der Substituent Hydroxy, Niedrigalkylthio, Amino, Niedrigalkylamino, Niedrigdialkylamino und Acylamino sein kann; substituiertes Niedrigalkyl der Formel $R_A(CH_2)_n$—Q—$(CH_2)$m—, worin n 0—2 ist, m 1—3 ist, $R_A$ Aryl oder Heteroaryl ist, das gegebenenfalls durch Alkyl, Halogen, Dihalogen, Amino, Cyano, Hydroxy oder Niedrigalkoxy substituiert ist, und Q O, S, N—$R_B$, $CONR_C$, $NR_CCO$ oder CH=CH ist, worin $R_B$ Wasserstoff, Niedrigalkyl, Aralkyl, Niedrigalkanoyl oder Aroyl ist und $R_C$ Wasserstoff oder Niedrigalkyl ist; Aralkyl oder Heteroaralkyl, welches verzweigte niedrige Alkylgruppen umfaßt, bedeutet.

3. Die Verbindung des Anspruchs 1, welche ein Glied aus der Gruppe:

4-N-(1(S)-Carboxyethyl)-6(R)-(1(S)-carboethoxy-3-phenylpropylamino)perhydro-1,4-thiazepin-5-on;

4-N-(1(S)-Carboxyethyl)-6(R)-(1(S)-carboxy-3-phenylpropylamino)perhydro-1,4-thiazepin-5-on;

4-N-Carboxymethyl-6(R)-(1(S)-carboethjoxy-3-phenylpropylamino)perhydro-1,4-thiazepin-5-on; und

4-N-Carboxymethyl-6(R)-(1(S)-carboxy-3-phenylpropylamino)perhydro-1,4-thiazepin-5-on ist.

4. Eine pharmazeutische Zusammensetzung, die zur Behandlung von Hypertension verwendbar ist, welche einen pharmazeutisch annehmbaren Träger und eine pharmazeutisch wirksame Menge einer Verbindung der Formel:

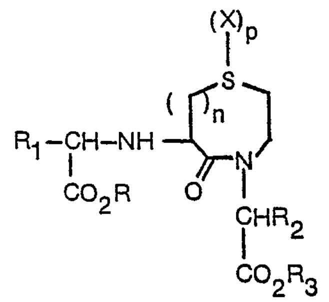

(I)

worin

R und $R_3$ unabhängig voneinander Wasserstoff, Niedrigalkyl, Aryl und Aralkyl sind;

$R_1$ Wasserstoff; Alkyl mit 1 bis 12 Kohlenstoffatomen, welches verzweigte, cyclishe und ungesättgite Alkylgruppen umfaßt; substituiertes Niedrigalkyl, worin der Substituent Halogen, Hydroxy, Carboxy, Niedrigalkylthio, Niedrigalkoxy, Niedrigalkoxycarbonyl, Niedrigaralkoxycarbonyl, Amino, Niedrigalkylamino, Niedrigdialkylamino oder Acylamino sein kann; substituiertes Niedrigalkyl mit der Formel $R_A(CH_2)_n$—Q—$(CH_2)_m$, worin n 0—2 ist, m 1—3 ist, $R_A$ Aryl oder Heteroaryl ist, das gegebenenfalls durch Amino, Niedrigialkylamino, Niedrigalkylamino, Hydroxy, Hydroxyniedrigalkyl, Aminoniedrigalkyl, Trihalogenniedrigalkyl, Cyano, Nitro, Sulfonamido, Aroyl, Niedrigalkyl, Halogen, Dihalogen und Niedrigalkoxy substituiert ist, und Q O, S, N—$R_B$, $CONR_C$, $NR_CCO$, CH=CH ist, worin $R_B$ Wasserstoff, Niedrigalkyl, Aryl, Aralkyl, Niedrigalkanoyl oder Aroyl ist, und $R_C$ Wasserstoff oder Niedrigalkyl ist; Aryl; substituiertes Aryl, worin der Substituent Niedrigalkyl, Aminoniedrigalkyl, Niedrigalkoxy, Aryloxy, Aroyl, Hydroxy, Halogen oder Dihalogen ist; Aralkyl oder Heteroaralkyl, welches verzweigte niedrige Alkylgruppen umfaßt; substituiertes Aralkyl oder substituiertes Heteroaralkyl, welches verzweigte niedrige Alkylgruppen unfaßt, worin die niedrigen Alkylgruppen durch Amino, Acylamino oder Hydroxyl substituiert sein können, und die Aryl- und Heteroarylgruppen durch Halogen, Dihalogen, Niedrigalkyl, Hydroxy, Niedrigalkoxy, Aryloxy, Aroyl, Arylthio, Amino, Aminoniedrigalkyl, Niedrigalkanoylamino, Aroylamino, Niedrigdialkylamino, Niedrigalkylamino, Hydroxy, Hydroxyniedrigalkyl, Trihalogenniedrigalkyl, Nitro, Cyano oder Sulfonamido substituiert sein können; bedeutet;

$R_2$ Wasserstoff, Niedrigalkyl ist;

n 1 oder 2 ist;

p 0 oder 1 ist;

X Sauerstoff ist; oder

die pharmazeutisch annehmbaren Salze davon umfaßt.

5. Die Zusammensetzung des Anspruchs 4, worin die genannte Verbindung ein Glied aus der Gruppe:

4-N-(1(S)-Carboxyethyl)-6(R)-(1(S)-carboethoxy-3-phenylpropylamino)perhydro-1,4-thiazepin-5-on;

4-N-(1(S)-Carboxyethyl)-6(R)-(1(S)-carboxy-3-phenylpropylamino)perhydro-1,4-thiazepin-5-on;

4-N-Carboxymethyl-6(R)-(1(S)-carboethoxy-3-phenylpropylamino)perhydro-1,4-thiazepin-5-on; und

4-N-Carboxymethyl-6(R)-(1(S)-carboxy-3-phenylpropylamino)perhydro-1,4-thiazepin-5-on ist.

6. Die Zusammensetzung des Anspruchs 4, welche eine antihypertensive und/oder diuretische Verbindung, ausgewählt aus der Gruppe bestehand aus Hydrochlorthiazid, Methyldopa, Timolol, dem Pivaloyloxyethylester von Methyldopa, Indacrinon und variablen Verhaltnissen seiner Enantiomere, (+)-4-{3-{[2-(1-Hydroxycyclohexyl)ethyl]-4-oxo-2-thiazolidinyl}propyl}benzoesäure sowie Gemischen und Kominationen davon, enthält.

7. Ein Verfahren zur Herstellung einer Verbindung der Formel:

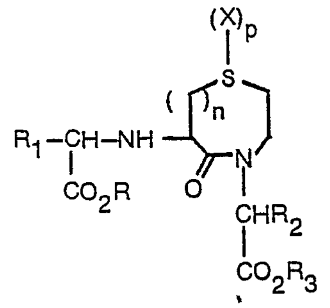

worin R, $R_1$, $R_2$, $R_3$, X, n und p wie in Anspruch 1 definiert sind, welches Verfahren die reduktive Kondensation einer $\alpha$-Ketosäure oder eines $\alpha$-Ketoesters der Formel $R_1CO—CO_2R$, worin R und $R_1$ wie in Anspruch 1 definiert sind, mit einem $\alpha$-Aminothialactam der Formel:

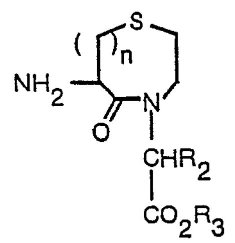

worin $R_2$, $R_3$ und n wie in Anspruch 1 definiert sind, unter Bildung, nach fakultativer Umsetzung mit einer Persäure und erforderlichenfalls Entfernung von Schutzgruppen, des gewünschten Produktes, und gewünschtenfalls die Isolierung des biologisch aktiveren Isomers durch Chromatographie oder fraktionierte Kristallisation und, falls weiterhin erwünscht, die Herstellung eines Salzes davon durch übliche Mittel, umfaßt.

## Patentansprüche für den Vertragsstaat: AT

1. Ein Verfahren zur Herstellung einer Verbindung der Formel:

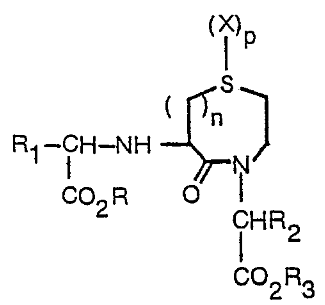

(I)

worin

R und $R_3$ unabhängig voneinander Wasserstoff, Niedrigalkyl, Aryl und Aralkyl sind;

$R_1$ Wasserstoff; Alkyl mit 1 bis 12 Kohlenstoffatomen, welches verzweigte, cyclische und ungesättigte Alkylgruppen umfaßt; substituiertes Niedrigalkyl, worin der Substituent Halogen, Hydroxy, Carboxy, Niedrigalkylthio, Niedrigalkoxy, Niedrigalkoxycarbonyl, Niedrigaralkoxycarbonyl, Amino, Niedrigalkylamino, Niedrigdialkylamino oder Acylamino sein kann; substituiertes Niedrigalkyl mit der Formel $R_A(CH_2)_n—Q—(CH_2)_m$, worin n 0—2 ist, m 1—3 ist, $R_A$ Aryl oder Heteroaryl ist, das gegebenenfalls durch Amino, Niedrigdialkylamino, Niedrigalkylamino, Hydroxy, Hydroxyniedrigalkyl, Aminoniedrigalkyl, Trihalogenniedrigalkyl, Cyano, Nitro, Sulfonamido, Aroyl, Niedrigalkyl, Halogen, Dihalogen und Niedrigalkoxy substituiert ist, und Q O, S, $N—R_B$, $CONR_C$, $NR_CCO$, CH=CH ist, worin $R_B$ Wasserstoff, Niedrigalkyl, Aryl, Aralkyl, Niedrigalkanoyl oder Aroyl ist, und $R_C$ Wasserstoff oder Niedrigalkyl ist; Aryl; substituiertes Aryl, worin der Substituent Niedrigalkyl, Aminoniedrigalkyl, Niedrigalkoxy, Aryloxy, Aroyl, Hydroxy, Halogen oder Dihalogen ist; Aralkyl oder Heteroaralkyl, welches verzweigte niedrige Alkylgruppen umfaßt; substituiertes Aralkyl oder substituiertes Heteroaralkyl, welches verzweitge niedrige Alkylgruppen unfaßt, worin die niedrigen Alkylgruppen durch Amino, Acylamino oder Hydroxyl substituiert sein können, und die Aryl- und Heteroarylgruppen durch Halogen, Dihalogen, Niedrigalkyl, Hydroxy, Niedrigalkoxy, Aryloxy, Aroyl, Arylthio, Amino, Aminoniedrigalkyl, Niedrigalkanoylamino, Aroylamino, Niedrigdialkylamino, Niedrigalkylamino, Hydroxy, Hydroxyniedrigalkyl, Trihalogenniedrigalkyl, Nitro, Cyano oder Sulfonamido substituiert sein können; bedeutet;

$R_2$ Wasserstoff, Niedrigalkyl ist;
n 1 oder 2 ist;
p 0 oder 1 ist;
X Sauerstoff ist; und
der pharmazeutisch annehmbaren Salze davon, welches Verfahren die reduktive Kondensation einer $\alpha$-Ketosäure oder eines $\alpha$-Ketoesters der Formel $R_1CO—CO_2R$, worin R und $R_1$ wie oben definiert sind, mit einem $\alpha$-Aminothialactam der Formel

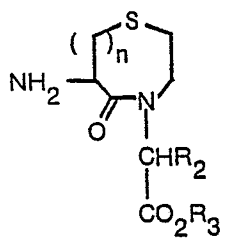

worin $R_2$, $R_3$ und n wie oben definiert sind, unter Bildung, nach fakultativer Umsetzung mit einer Persäure und erforderlichenfalls Entfernung von Schutzgruppen, des gewünschten Produktes, und gewünschtenfalls die Isolierung des biologisch aktiveren Isomers durch Chromatographie oder fraktionierte Kristallisation und, falls weiterhin erwünscht, die Herstellung eines Salzes davon durch übliche Mittel, umfaßt.

2. Das Verfahren des Anspruchs 1 zur Herstellung einer Verbindung gemäß Anspruch 1, worin

$R_1$ Alkyl mit 1 bis 10 Kohlenstoffatomen, welches verzweigte, cyclische und ungesättigte Alkylgruppen umfaßt; substituiertes Niedrigalkyl, worin der Substituent Hydroxy, Niedrigalkylthio, Amino, Niedrigalkylamino, Niedrigdialkylamino und Acylamino sein kann; substituiertes Niedrigalkyl der Formel $R_A(CH_2)_n—Q—(CH_2)m—$, worin n 0—2 ist, m 1—3 ist, $R_A$ Aryl oder Heteroaryl ist, das gegebenenfalls durch Alkyl, Halogen, Dihalogen, Amino, Cyano, Hydroxy oder Niedrigalkoxy substituiert ist, und Q O, S, $N—R_B$, $CONR_C$, $NR_CCO$ oder CH=CH ist, worin $R_B$ Wasserstoff, Niedrigalkyl, Aralkyl, Niedrigalkanoyl oder Aroyl ist und $R_C$ Wasserstoff oder Niedrigalkyl ist; Aralkyl oder Heteroaralkyl, welches verzweigte niedrige Alkylgruppen umfaßt, bedeutet.

3. Das Verfahren des Anspruchs 1 zur Herstellung einer Verbindung gemaß Anspruch 1, welche ein Glied aus der Gruppe:
4-N-(1(S)-Carboxyethyl)-6(R)-(1(S)-carboethoxy-3-phenylpropylamino)perhydro-1,4-thiazepin-5-on;
4-N-(1(S)-Carboxyethyl)-6(R)-(1(S)-carboxy-3-phenylpropylamino)perhydro-1,4-thiazepin-5-on;
4-N-Carboxymethyl-6(R)-(1(S)-carboethoxy-3-phenylpropylamino)perhydro-1,4-thiazepin-5-on; und
4-N-Carboxymethyl-6(R)-(1(S)-carboxy-3-phenylpropylamino)perhydro-1,4-thiazepin-5-on ist.

**Revendications pour les Etats contractants: BE CH DE FR GB IT LI LU NL SE**

1. Composé de formule:

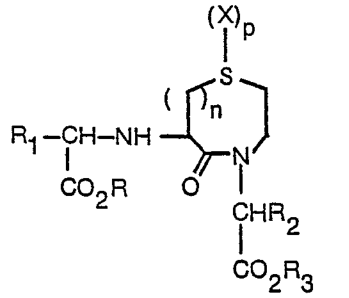

(I)

où
R et $R_3$ sont indépendamment un hydrogène, un alkyle inférieur, un aryle et un aralkyle;

$R_1$ est un hydrogène; un alkyle de 1 à 12 atomes de carbone qui comprend les groupes alkyles ramifiés, cycliques et insaturés; un alkyle inférieur substitué où le substituant peut être un halogéno, un hydroxy, un carboxy, un alkyl inférieur thio, un alcoxy inférieur, un alcoxy-inférieur carbonyle, un aralcoxy inférieur carbonyle un amino, un alkyl inférieur amino, un dialkyl inférieur amino ou un acylamino; un alkyle inférieur substitué ayant pour formule $R_A(CH_2)_n—Q—(CH_2)_m$ où n est 0—2, m est 1—3, $R_A$ est un aryle ou un hétéroaryle éventuellement substitué par un amino, un dialkyl inférieur amino, un alkylinférieur amino, un hydroxy, un hydroxyalkyle inférieur, un aminoalkyle inférieur, un trihalogénoalkyle inférieur, un cyano, un nitro, un sulfonamido, un aroyle, un alkyle inférieur, un halogéno, un dihalogéno et un alcoxy inférieur, et Q est O, S, $N—R_B$, $CONR_C$, $NR_CCO$, CH=CH où $R_B$ est un hydrogène, un alkyle inférieur, un aryle, un aralkyle, un alcanoyle inférieur ou un aroyle, et $R_C$ est un hydrogène ou un alkyle inférieur; un aryle; un aryle substitué dont le substituant est un alkyle inférieur, un aminoalkyle inférieur, un alcoxy inférieur, un aryloxy, un aroyle, un hydroxy, un halogéno ou un dihalogéno; un aralkyle ou un hétéro-aralkyle comprenant des groupes alkyles inférieurs ramifiés; un aralkyle substitué ou un hétéro-aralkyle substitué comprenant des groupes alkyles inférieurs ramifiés où les groupes alkyles inférieurs peuvent être substitués par un amino, un acylamino ou un hydroxy, et les groupes aryles ou hétéroaryles peuvent être substitués par un halogéno, un dihalogéno, un alkyle inférieur, un hydroxy, un alcoxy inférieur, un aryloxy, un aroyle, un arylthio, un amino, un amino-alkyle inférieur, un alcanoyl inférieur amino, un aroylamino, un dialkyl inférieur amino, un alkyl inférieur amino, un hydroxy, un hydroxyalkyle inférieur, un trihalogénoalkyle inférieur, un nitro, un cyano ou un sulfonamido;

$R_2$ est un hydrogène, un alkyle inférieur;

n est 1 ou 2;

p est 0 ou 1;

X est un oxygène; et, leurs sels acceptables en pharmacie.

2. Composé selon la revendication 1, où

$R_1$ est un alkyle de 1 à 10 atomes de carbone qui comprend des groupes alkyles ramifiés, cycliques et insaturés; un alkyle inférieur substitué où le substituant peut être un hydroxy, un alkyl inférieur thio, un amino, un alkyl inférieur amino, un dialkyl inférieur amino et un acylamino; un alkyle inférieur substitué ayant pour formule $R_A(CH_2)_n—Q—(CH_2)_m—$ où n est 0—2, m est 1—3, $R_A$ est un aryle ou un hétéroaryle éventuellement substitué par un alkyle, un halogéno, un dihalogéno, un amino, un cyano, un hydroxy ou un alcoxy inférieur, et Q est O, S, $N—R_B$, $CONR_C$, $NR_CCO$, ou CH=CH où $R_B$ est un hydrogène, un alkyle inférieur, un aralkyle, un alcanoyle inférieur ou un aroyle, et $R_C$ est un hydrogène ou un alkyle inférieur; un aralkyle ou un hétéroaralkyle comprenant des groupes alkyles inférieurs ramifiés.

3. Composé de la revendication 1 qui est un membre du groupe:

4-N-(1(S)-carboxyéthyl)-6(R)-(1(S)-carboéthoxy-3-phénylpropylamino)perhydro-1,4-thiazépine-5-one;

4-N-(1(S)-carboxyéthyl)-6(R)-(1(S)-carboxy-3-phénylpropylamino)perhydro-1,4-thiazépine-5-one;

4-N-carboxyméthyl-6(R)-(1(S)-carbo-éthoxy-3-phénylpropylamino)perhydro-1,4-thiazépine-5-one; et

4-N-carboxyméthyl-6(R)-(1(S)-carboxy-3-phénylpropyl-amino)perhydro-1,4-thiazépine-5-one.

4. Composition pharmaceutique utile dans le traitement de l'hypertension qui comprend un support acceptable en pharmacie et une quantité pharmaceutique efficace d'un composé de formule

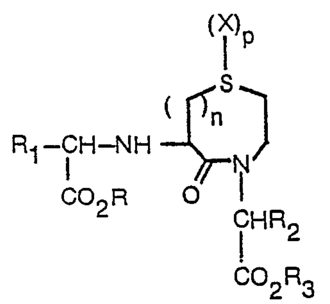

(I)

où

R et $R_3$ sont indépendamment un hydrogène, un alkyle inférieur, un aryle et un aralkyle;

$R_1$ est un hydrogène; un alkyle de 1 à 12 atomes de carbone qui comprend des groupes alkyles ramifiés, cycliques et insaturés; un alkyle inférieur substitué où le substituant peut être un halogéno, un hydroxy, un carboxy, un alkyl inférieur thio, un alcoxy inférieur, un alcoxy inférieur carbonyle, un aralcoxyinférieur carbonyle, un amino, un alkyl inférieur amino, un dialkyl inférieur amino ou un acylamino; un alkyle inférieur substitué ayant pour formule $R_A(CH_2)_n—Q—(CH_2)_m$ où n est 0—2, m est 1—3, $R_A$ est un aryle ou un hétéroaryle éventuellement substitué par un amino, un dialkyl inférieur amino un alkylinférieur amino, un hydroxy, un hydroxyalkyle inférieur, un aminoalkyle inférieur, un trihalogénoalkyle inférieur, un cyano, un nitro, un sulfonamido, un aroyle, un alkyle inférieur, un halogéno, un dihalogéno, et un alcoxy inférieur, et Q est O, S, $N—R_B$, $CONR_C$, $NR_CCO$, CH=CH où $R_B$ est un hydrogène, un alkyle inférieur, un aryle, un aralkyle, un alcanoyle inférieur ou un aroyle, et $R_C$ est un hydrogène ou un alkyle inférieur; un aryle; un aryle substitué dont le substituant est un alkyle inférieur, un aminoalkyle inférieur, un alcoxy inférieur, un aryloxy, un aroyle, un hydroxy, un halogéno ou un

dihalogéno; un aralkyle ou un hétéroaralkyle comprenant des groupes alkyles inférieurs ramifiés; un aralkyle substitué ou un hétéroaralkyle substitué comprenant des groupes alkyles inférieurs ramifiés où les groupes alkyles inférieurs peuvent être substitués par un amino, un acylamino ou un hydroxy, et les groupes aryles ou hétéroaryles peuvent être substitués par un halogéno, un dihalogéno, un alkyle inférieur, un hydroxy, un alcoxy inférieur, un aryloxy, un aroyle, un arylthio, un amino, un aminoalkyle inférieur, un alcanoyl inférieur amino, un aroylamino, un dialkyl inférieur amino, un alkylinférieur amino, un hydroxy, un hydroxyalkyle inférieur, un trihalogénoalkyle inférieur, un nitro, un cyano ou un sulfonamido;

$R_2$ est un hydrogène, un alkyle inférieur;

n est 1 ou 2;

p est 0 ou 1;

X est un oxygène; et, leurs sels acceptables en pharmacie.

5. Composition selon la revendication 4, où ledit composé est un membre du groupe:
4-N-(1(S)-carboxyéthyl)-6(R)-(1(S)-carbo-éthoxy-3-phényl-propylamino)perhydro-1,4-thiazépine-5-one;
4-N-(1(S)-carboxyéthyl)-6(R)-(1(S)-carboxy-3-phénylpropylamino)perhydro-1,4-thiazépine-5-one;
4-N-carboxyméthyl-6(R)-(1(S)-carbo-éthoxy-3-phényl-propylamino)perhydro-1,4-thiazépine-5-one; et,
4-N-carboxyméthyl-6(R)-(1(S)-carboxy-3-phénylpropylamino)perhydro-1,4-thiazépine-5-one.

6. Composition selon la revendication 4, qui comprend un composé antihypertenseur et/ou diurétique choisi dans le groupe constitué par l'hydrochlorothiazide, la méthyldopa. le timolol, l'ester pivaloyloxyéthylique de la méthyldopa, l'indacrinone et des rapports variables de ses énantiomères, l'acide (+)-4-{3-{[2-(1-hydroxycyclohexyl)éthyl]-4-oxo-2-thiazolidinyl}propyl}benzoïque, ainsi que leurs mélanges et combinaisons.

7. Procédé de préparation d'un composé ayant pour formule:

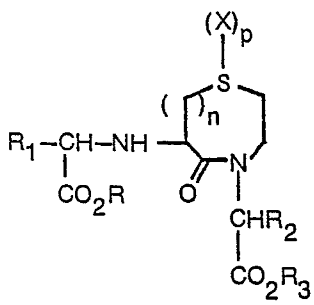

où R, $R_1$, $R_2$, $R_3$, X, n et p sont comme défini dans la revendication 1, lequel procédé comprend: la condensation par réduction d'un $\alpha$-cétoacide ou d'un $\alpha$-cétoester de formule $R_1CO—CO_2R$ où R et $R_1$ sont comme défini dans la revendication 1 avec un $\alpha$-aminothialactame de formule:

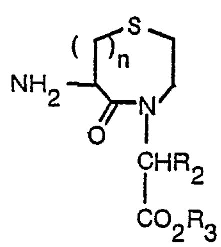

où $R_2$, $R_3$ et n sont comme défini dans la revendication 1 pour fournir, éventuellement après réaction avec un peracide, puis élimination des groupes protecteurs, s'il est nécessaire, le produit désiré et, si on le désire, l'isolement de l'isomère ayant le plus d'activité biologique par chromatographie ou cristallisation fractionnée et, de plus, si on le désire, la préparation d'un sel correspondant de façon classique.

**Revendications pour l'Etat contractant: AT**

1. Procédé de préparation d'un composé ayant pour formule:

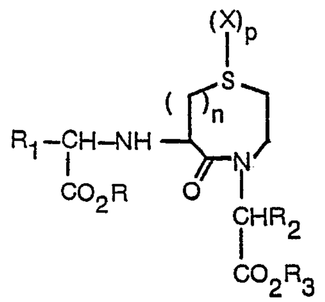

(I)

où

R et $R_3$ sont indépendamment un hydrogène, un alkyle inférieur, un aryle et un aralkyle;

$R_1$ est un hydrogène; un alkyle de 1 à 12 atomes de carbone qui comprend des groupes alkyles ramifiés, cycliques et insaturés; un alkyle inférieur substitué où le substituant peut être un halogéno, un hydroxy, un carboxy, un alkylinférieur thio, un alcoxy inférieur, un alcoxyinférieur carbonyle, un aralcoxy inférieur carbonyle un amino, un alkyl inférieur amino, un dialkyl inférieur amino ou un acylamino; un alkyle inférieur substitué ayant pour formule $R_A(CH_2)_n$—Q—$(CH_2)_m$ où n est 0—2, m est 1—3, $R_A$ est un aryle ou un hétéroaryle éventuellement substitué par un amino, un dialkyl inférieur amino, un alkylinférieur amino, un hydroxy, un hydroxyalkyle inférieur, un aminoalkyle inférieur, un trihalogénoalkyle inférieur, un cyano, un nitro, un sulfonamido, un aroyle, un alkyle inférieur, un halogéno, un dihalogéno et un alcoxy inférieur, et Q est O, S, N—$R_B$, $CONR_C$, $NR_CCO$, CH=CH où $R_B$ est un hydrogène, un alkyle inférieur, un aryle, un aralkyle, un alcanoyle inférieur ou un aroyle, et $R_C$ est un hydrogène ou un alkyle inférieur; un aryle; un aryle substitué dont le substituant est un alkyle inférieur, un aminoalkyle inférieur, un alcoxy inférieur, un aryloxy, un aroyle, un hydroxy, un halogéno ou un dihalogéno; un aralkyle ou un hétéro-aralkyle comprenant des groupes alkyles inférieurs ramifiés; un aralkyle substitué ou un hétéroaralkyle substitué comprenant des groupes alkyles inférieurs ramifiés où les groupes alkyles inférieurs peuvent être substitués par un amino, un acylamino ou un hydroxy, et les groupes aryles ou hétéroaryles peuvent être substitués par un halogéno, un dihalogéno, un alkyle inférieur, un hydroxy, un alcoxy inférieur, un aryloxy, un aroyle, un arylthio, un amino, un aminoalkyle inférieur, un alcanoyl inférieur amino, un aroylamino, un dialkyl inférieur amino, un alkylinférieur amino, un hydroxy, un hydroxyalkyle inférieur, un trihalogénoalkyle inférieur, un nitro, un cyano ou un sulfonamido;

$R_2$ est un hydrogène, un alkyle inférieur;

n est 1 ou 2;

p est 0 ou 1;

X est un oxygène; et, leurs sels acceptables en pharmacie, lequel procédé comprend: la condensation par réduction d'un $\alpha$-céto-acide ou d'un $\alpha$-cétoester ayant pour formule $R_1CO$—$CO_2R$ où R et $R_1$ sont comme défini ci-dessus avec un $\alpha$-aminothialactame de formule:

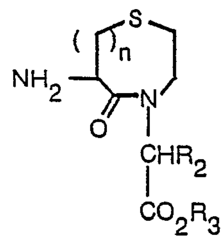

où $R_2$, $R_3$ et n sont comme défini ci-dessus, pour fournir, éventuellement après réaction avec un peracide suivie de l'élimination des groupes protecteurs, s'il est nécessaire, le produit désiré et, si on le désiré, l'isolement de l'isomère ayant le plus d'activité biologique par chromatographie ou cristallisation fractionnée et, de plus, si on le désire, la préparation d'un sel correspondant de façon classique.

2. Procédé selon la revendication 1 pour préparer un composé selon la revendication 1, où $R_1$ est un alkyle de 1—10 atomes de carbone qui comprend des groupes alkyles ramifiés, cycliques et insaturés; un alkyle inférieur substitué dont le substituant peut être un hydroxy, un alkyl inférieur thio, un amino, un alkyl inférieur amino, un dialkyl inférieur amino et un acylamino; un alkyle inférieur substitué ayant pour formule $R_A(CH_2)_n$—Q—$(CH_2)_m$— où n est 0—2, m est 1—R, $R_A$ est un aryle ou un hétéroaryle éventuellement substitué par un alkyle, un halogéno, un dihalogéno, un amino, un cyano, un hydroxy ou un alcoxy inférieur, et Q est O, S, N—$R_B$, $CONR_C$, $NR_CCO$, ou CH=CH où $R_B$ est un hydrogène, un alkyle inférieur, un aralkyle, un alcanoyle inférieur ou un aroyle, et $R_C$ est un hydrogène ou un

alkyle inférieur; un aralkyle ou un hétéroaralkyle comprenant des groupes alkyles inférieurs ramifiés.

3. Procédé selon la revendication 1 pour préparer composé selon la revendication 1 qui est un membre du groupe:
4-N-(1(S)-carboxyéthyl)-6(R)-(1(S)-carboéthoxy-3-phénylpropylamino)perhydro-1,4-thiazépine-5-one;
4-N-(1(S)-carboxyéthyl)-6(R)-(1(S)-carboxy-3-phénylpropylamino)perhydro-1,4-thiazépine-5-one;
4-N-carboxyméthyl-6(R)-(1(S)-carboéthoxy-3-phénylpropylamino)perhydro-1,4-thiazépine-5-one; et
4-N-carboxyméthyl-6(R)-(1(S)-carboxy-3-phénylpropyl-amino)perhydro-1,4-thiazépine-5-one.